# EUROPEAN PATENT SPECIFICATION

(11) **EP 0 472 526 B1**
(45) Date of publication and mention of the grant of the patent: **08.05.1996**
(21) Application number: 90905307.6
(22) Date of filing: 23.03.1990
(51) Int. Cl.: C07D 451/00, A61K 31/46

(54) **5-AMINOCARBONYL-5H-DIBENZO a,d]CYCLOHEPTEN-5,10-IMINES FOR TREATMENT OF EPILEPSY AND COCAINE ADDICTION**
5-AMINOCARBONYL-5H-DIBENZO-A,D]CYCLOHEPTEN-5,10-IMINE ZUR BEHANDLUNG VON EPILEPSIE UND KOKAINSUCHT
5-AMINOCARBONYLE-5H-DIBENZO a,d]CYCLOHEPTENE-5,10-IMINES POUR LE TRAITEMENT DE L'EPILEPSIE ET DE LA COCAINOMANIE

(30) Priority: 09.05.1989 US 349187
(43) Date of publication of application: 04.03.1992
(73) Proprietor: THE UNITED STATES OF AMERICA as represented by the Secretary United States Department of Commerce, Springfield, Virginia 22161 (US)
(72) Inventor: MONN, James, A., Indianapolis, IN 46254 (US); THURKAUF, Andrew, Crofton, MD 21114 (US); YAMAGUCHI, Shunichi, Bethesda, MD 20892 (US); ROGAWSKI, Michael, A., Columbia, MD 21046 (US); RICE, Kenneth, C., Bethesda, MD 20892 (US); MATTSON, Mariena, V., Rockville, MD 20851 (US); JACOBSON, Arthur, E., Potomac, MD 20854 (US)
(74) Representative: Nash, David Allan
(86) International application number: US9001502
(87) International publication number: WO9013297

(56) References cited:
- EP-A- 0 264 183
- US-A- 4 009 273
- US-A- 4 399 141
- TETRAHEDRON LETTERS, vol. 30, no. 8, 1989, pages 911-914, Oxford, GB; J.A. MONN et al.: "A bridgehead alpha-amino carbanion: facile preparation of C5(bridgehead)-substituted analogues of (+)-5H-dibenzo[a,d]cyclohepten-5,10-imine including a stable alpha-iodo secondary amine"
- JOURNAL OF MEDICINAL CHEMISTRY, vol. 33, no. 3, March 1990, pages 1069-1076, Washington, DC, US; J.A. MONN et al.: "Synthesis and structure-activity relationship of C5-substituted analogues of (+)-10,11-dihydro-5H-dibenzo[a, d]cyclohepten-5,10-imine [(+)-desmethyl-MK801]: ligands for the NMDA receptor- coupled phencyclidine binding site"
- CHEMICAL ABSTRACTS, VOL. 97, 1982, pages 62-63, CLINESCHMIDT et al., 1982 Abstract 16955d.
- J. MED. CHEM., 1990, Vol. 33, 1047-1052.

## Description

This invention is in the field of clinical neurology and relates specifically to compounds, compositions and methods for treatment of patients with generalized epilepsy or partial (symptomatic) epilepsy. This invention also relates to compounds, compositions and methods of treatment for drug craving in patients addicted to cocaine.

Epilepsy is a symptom of excessive temporary neuronal discharge, due to intracranial or extracranial causes; it is characterized clinically by discrete episodes, which tend to be recurrent, in which there is a disturbance of movement, behavior, perception and/or conciousness. The precise mechanism involved in the excessive neuronal discharge of epilepsy remains unknown. Nerve tissue is electronically excitable and this excitability is influenced by many factors. The relative tendency of individuals to experience excessive neuronal discharge leading to seizures is referred to as the seizure threshold of the brain. Normal individuals possessing a high threshold never experience abnormal periods of neuronal activity. Individuals with low threshold will periodically experience those episodes associated with epileptic seizures. The causes of the seizures may be from lowered neuronal resting potential due to inherent abnormalities in cellular ion gradients or in excitatory and inhibitory neuronal transmitter systems. Seizure spread may also be potentiated by damage to inhibitory neurons due to traumatic, infective, vascular or neoplastic causes. Hyperexcitability of neurons can also be a chronic effect caused by pyrexia, hypoxia, hypoglycemia, overhydration, alkalosis, withdrawal of barbituates or alcoholism. In addition, seizures may be induced by convulsant drugs, electric shock, auditory or visual stimulus and physical and emotional stress.

Pharmaceutical agents used for the control of epilepsy fall into a variety of chemical classes including, but not limited to, acridines, amphetamines, barbiturates, carbamates, benzodiazepines, butyric acid derivatives, glutamic acid derivatives, valproic acid derivatives, ureas, hydantoins, oxazolidinediones, succinimides, sulfonamides and hydrazones [see J.A.Vida, "Anticonvulsants", Academic Press, New York, 1977]. Convulsant seizures have been found to originate locally (at primary focii) in the brain and spread to other regions. The mode of action of most anticonvulsant drugs involves either the suppression of preconvulsant stimulus at the primary focus or inhibition of the spread of the excessive electrical activity to other brain regions [see.F. Morrell, W. Bradley and M. Ptashe, Neurology, 9, 492 (1959)]. The majority of clinically useful anticonvulsant have a cyclic ureide structure.

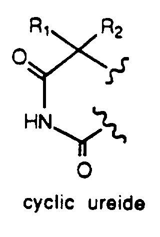

These include the important drugs phenobarbital and diphenylhydantoin (Dilantin) Other clinically important compounds which do not possess the cyclic ureide structure are primidone, benzodiazepines and carbamazepine.

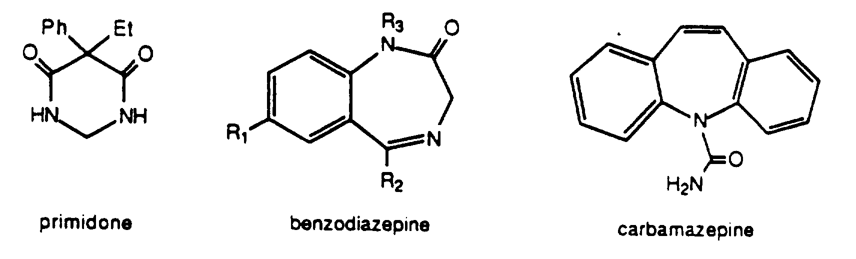

Carbamazepine (5-carbamyl-5H-dibenzo[b,f]azepine) is a major anticonvulsant drug for the treatment of complex partial and generalized tonic-clonic seizures. Carbamazepine is often used in patients who have not responded satisfactorily to treatment with other agents. It shows good activity and low acute and motor toxicity. Although it has been implicated in bone marrow suppression only one case of toxic overdose has been reported. The low toxicity of carbamazepine may be due to its low bioavailability.

Recent studies have indicated that carbamazepine may possess the ability to restrict cocaine craving in cocaine addicts. In one study 59% of the addicts taking the medication were able to abstain from cocaine for a prescribed period compared to 17% who received a placebo.

Recently, MK-801, a new anticonvulsant of novel structure, has shown potential usefulness for seizures of focal origin and major generalized seizures. MK-801 is essentially free of the usual sedative side effects common to most of the commonly prescribed anticonvulsants [Clineschmidt *et al* Drug Dev. Res. 2, 123 (1982)]. Psychological disturbances in some of the patients in the clinical trials may be a consequence of the high affinity of the drug for phencyclidine binding sites in the central nervous system.

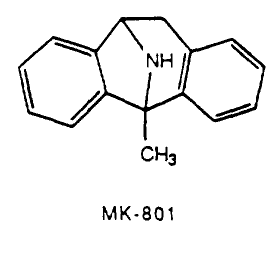

It has now been found that control of epileptic seizures and diminishment of drug craving in cocaine addicts is provided by treatment with an effective amount of a compound of the class of 5-aminocarbonyl-5H-dibenzo[a,d]cyclohepten-5,10-imines represented by Formula I: 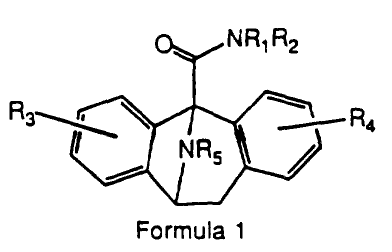 wherein each of R₁ and R₂ is independently selected from hydrogen, linear or branched alkyl groups of from one to twenty carbon atoms, alkenyl groups from two to twenty carbon atoms, alkynyl groups from two to twenty carbon atoms, cycloalkyl groups of three to eight carbon atoms, cycloalkenyl grouos from three to eight carbon atoms, and wherein R₁ and R₂ may be taken together to form a N-containing cyclic structure having two to eight carbon atoms, any of the said groups being optionally substituted with one or more substituents selected from alkyl, haloalkyl, hydroxyalkyl, alkenyl, oxo, hydroxyl, alkoxy, thio, alkoxyalkyl, amino, halo, cyano or mercapto, and wherein R₃ and R₄ is independently selected from hydrogen, halo, linear or branched alkyl groups of from one to ten carbon atoms, alkenyl groups from two to ten carbon atoms, alkynyl groups from two to ten carbon atoms, hydroxyl, amino, alkylamino, alkoxy, cyano, nitro, haloalkyl and mercapto, and wherein R₅ is selected from hydrogen, linear or branched alkyl groups of from one to ten carbon atoms, alkenyl groups from two to ten carbon atoms, alkynyl groups from two to ten carbon atoms, hydroxyl, phenyl, halcalkyl, aminoalkyl, 1-phenylmethyl, 2-phenylethyl and alkoxy, and wherein R₁ and R₅ taken together form a cyclic structure containing two nitrogen atoms possessing from two to six carbon atoms, any of the said groups being optionally substituted by alkyl, oxo, thio, alkoxy, hydroxy, amino, alkylamino, phenyl, haloalkyl and thio; or a pharmaceutically acceptable salt thereof.

A preferred class of compounds within Formula I are those wherein each of R₁ and R₂ is independently selected from hydrogen, alkyl, alkenyl, alkoxy or phenyl; wherein each of R₃ and R₄ is independently selected from hydrogen, alkyl, alkenyl, halo, haloalkyl, hydroxy, alkoxy, nitro, cyano, thio, mercapto, amino, alkylamino, wherein R₅ is selected from hydrogen, alkyl, alkenyl, haloalkyl, hydroxy, alkoxy, phenyl and aminoalkyl.

The term hydrido or hydrogen as used herein denotes a single hydrogen atom (H) which may be attached, for example, to a carbon atom or to a nitrogen atom to form a primary or secondary amino group. Where the term 'alkyl' is used, either alone or within other terms such as 'haloalkyl' or alkylamino' the term 'alkyl' embraces linear or branched radicals having one to ten carbon atoms. Preferred alkyl radicals are "lower alkyl" radicals having from one to five carbon atoms. The term 'cycloalkyl' embraces radicals having from three to ten carbon atoms, such as cyclopropyl and cyclobutyl. The term "haloalkyl" embraces radicals wherein one or more of the alkyl carbon atoms is substituted with one or more halogen atoms, preferably selected from fluoro, chloro and bromo. Specifically embraced by the term 'haloalkyl' are mononaloalkyl, dihaloalkyl and polyhaloalkyl groups. Examples of a polyhaloalkyl are trifluoromethyl, 2,2,2-trifluoroethyl and perfluoroethyl. The term 'alkenyl' embraces linear or branched radicals having from two to ten carbon atoms and containing at least one double bond. The term 'alkynyl' embraces linear or branched radicals having from two to ten carbon atoms containing at least one carbon-carbon triple bond. The term 'alkoxy' embraces linear or branched oxy-containing radicals having alkyl portions of from one to ten carbon atoms, such as methoxy group. The alkoxy radicals may be further substituted with one or more halo atoms, such as fluoro, chloro or bromo to provide haloalkoxy groups. The term 'alkylamino' embraces linear or branched nitrogen containing radicals where the nitrogen atom may be substituted with from one to three alkyl radicals of from one to ten carbon atoms, such as N-methylamino and N,N-dimethylamino.

Specific examples of alkyl groups are methyl, ethyl, propyl, isopropyl, n-butyl, isobutyl, tert-butyl, sec-butyl, neopentyl and n-pentyl. Typical alkenyl groups may have one unsaturated double bond, such as allyl or may have a plurality of double bonds.

Included within the family of compounds of Formula I are the tautomeric forms of the described compounds, isomeric forms such as diastereomers, and the pharmaceutically acceptable salts thereof. Examples of acids which may be employed to form pharmaceutically acceptable acid addition salts include such inorganic acids as hydrochloric acid, sulphuric acid and phosphoric acid and such organic acids as maleic acid, succinic acid and citric acid.

Compounds of Formula 1 may be prepared in accordance with the following general procedures:

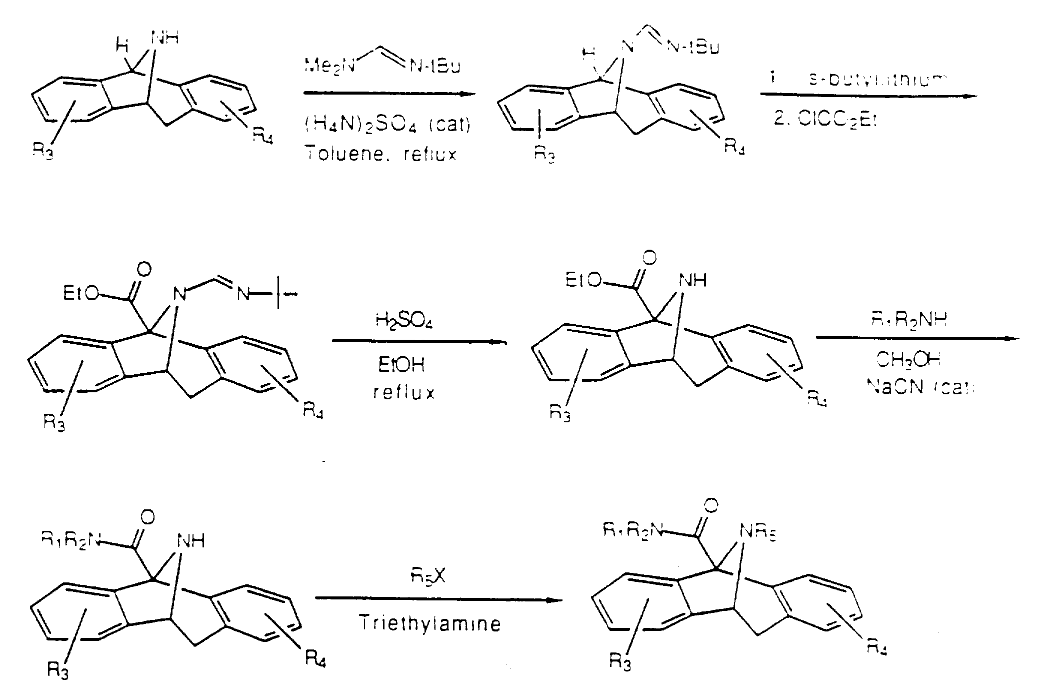

With reference to the foregoing scheme, the known and/or readily accessible racemic or optically active C5-unsubstituted-10,11-dihydro-5H-dibenzo[a,d]cyclohepten-5,10-imines are convened into their N-*tert*-butylformamidine derivatives. This is conveniently accomplished by reaction with a commercially available reagent, N'-tert-butyl-N,N-dimethylformamidine at elevated temperatures (110 °C) in the presence of an acid catalyst, generally ammonium sulfate. Secondly, the N-*tert*-butylformamidine derivatives in an anhydrous ethereal solvent at room temperature or at 5 °C are treated with *sec*-butyllithium followed by ethyl chloroformate. This results in the formation of the C5-substituted ethyl ester. The next step in the chemical sequence is the removal of the tert-butylformamidine moiety from the nitrogen atom of the parent ring system. This is achieved by heating in ethanolic sulfuric acid, and gives rise to 5-ethoxycarbonyl-10,11-dihydro-5H-dibenzo[a,d]cycloheptene-5,10-imines. The final step in the chemical synthesis is the replacement of ester functionality with an amide group. This is accomplished by warming the ester in methanol with the appropriate amine derivative. The presence of a catalytic amount of sodium cyanide facultes this reaction. If N-substitution is desired, the secondary amine is allowed to react with the appropriate alkyl halide in the presence of a suitable base (e.g. triethylamine).

### Example: Preparation of 5-Aminocarbonyl-10,11-dihydro-5H-dibenzo[a,d]cyclohepten-5,10-imine.

A mixture of 10,11-dihydro-5H-dibenzo[a,d]cyclohepten-5,10-mine (5.18 g, 25.0 mmol), N'-tert-butyl-N,N-dimethylformamidine (12.84 g, 100.0 mmol) arc a few crystals of ammonium sulfate in anhydrous toluene was warmed under reflux for 6 days. Evaporation of the solvent and purification of tne crude product by column chromatography employing 7% triethylamine in hexanes as the eluent afforded the N-*tert*-butylformamidinyl-10-11-dihydro-5H-dibenzo[a,d]cyclohepten-5,10-imine (6.98 g, 24,1 mmol, 96%): mp 63.64°.

A solution of this material (5,80 g, 20.0 mmol) in anhydrous ethyl ether (150 mL) under an atmosphere of nitrogen was treated at 5 °C with a 1.25 M solution of *sec*-butyllithium in cylohexane (20.0 mL, 25 mmol). The deep red colored solution of the anion was allowed to stir at this temperature for 40 minutes, then was treated with ethyl chloroformate (2.40 mL, 25.0 mmol). The solution color immediately changed to pale yellow, and gas chromatographic analysis of the reaction mixture demonstrated the complete consumption of the starting material. The reaction mixture was treated with ethanol (100 mL) and H₂SO₄ (0.56 mL. 10.0 mmol), and the ether was evaporated under reduced pressure. The etnanolic solution was warmed under reflux for 4 h, then was diluted with 0.5 N HCl (100 mL) and extracted with Et₂O (3 x 100). The aqueous part was made alkaline by addition 1 N NaOH, and extracted with Et₂O (3 x 100). The combined organic part was washed once with H₂O (100 mL), then dried over K₂CO₃ and concentrated to dryness affording 5-ethoxycarbonyl-10,11-dihydro-5H-dibenzo[a,d]cyclohepten-5,10-imine (3.37 g, 12.1 mmol, 60%). The hydrogen chloride salt was formed by passing a stream of anhydrous HCI gas through an ethereal solution of the secondary amine: mp 229 - 230 °C.

A solution of the preceding amino ester (0.53 g, 1.90 mmol) and sodium cyanide (10 mg) in anhydrous methanol (40 mL) which had been previously saturated at 5 °C with ammonia gas was warmed to 60 °C in a sealed tube for 40 h. After cooling to 5 °C, the solid which had formed was filtered, wasned with H₂O, and air-dried affording 5-aminocarbonyl-10,11-dihydro-5H-dibenzo[a,d]cyclohepten-5,10-imine (0.25 g, 1.0 mmol). The filtrate was extracted with CH₂Cl₂ (3 x 50), the organic pool was dned (K₂CO₃) and evaporated under reduced pressure, affording an additional quantity of the title compound (0.19 g, 0.76 mmol). Recrystallization of the combined samples from ethanol then gave the analytically pure material (0.37 g, 1.5 mmol, 78%). mp 235 - 236 °C.

Table I is comprised of a list of 20 specific compounds of most interest within formula I. The preparation of compound 1 in Table I is described in detail in the previous example. Compounds 2 through 20 may likewise be prepared in accordance with the above-described general synthesis procedures.

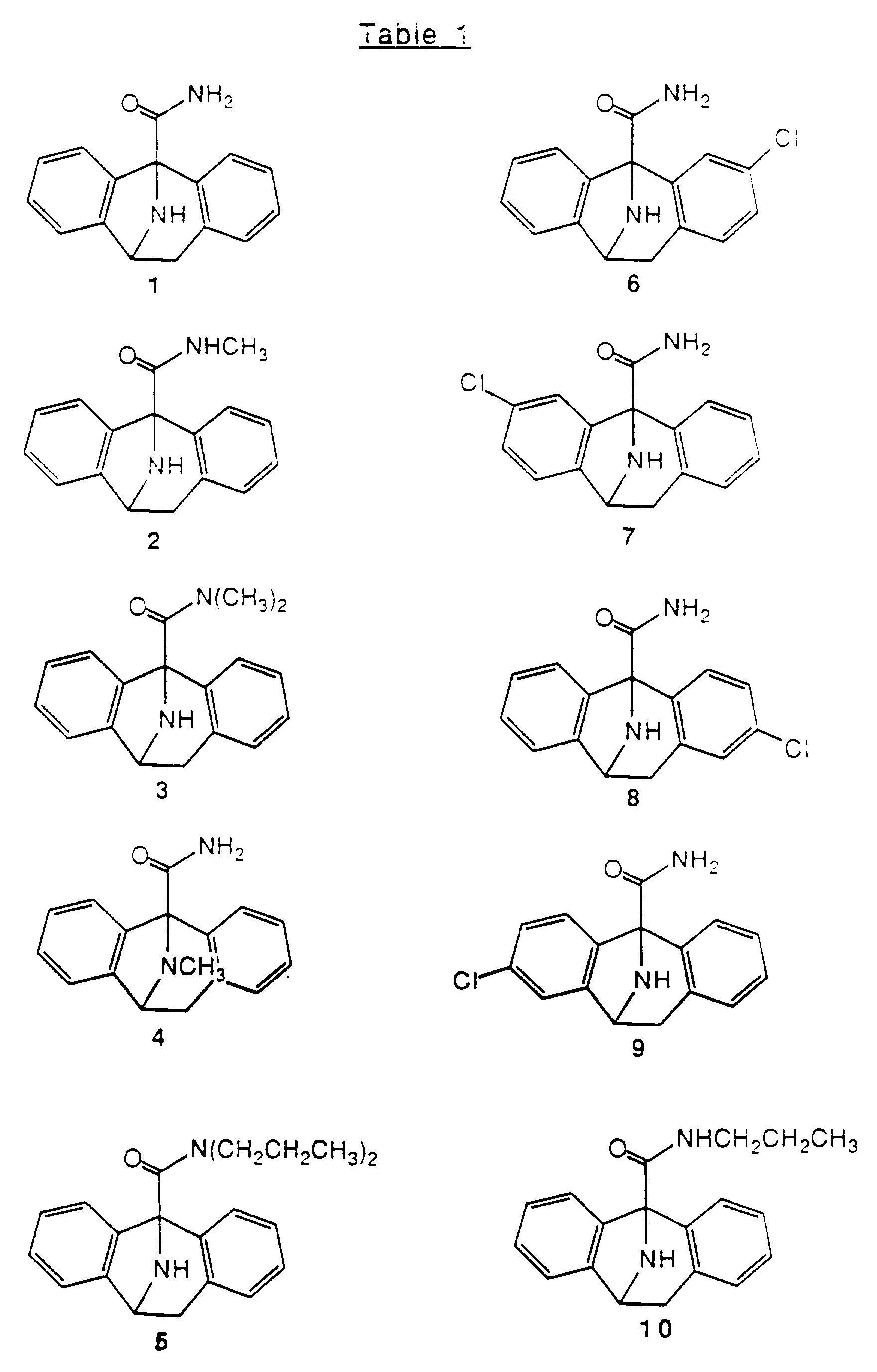

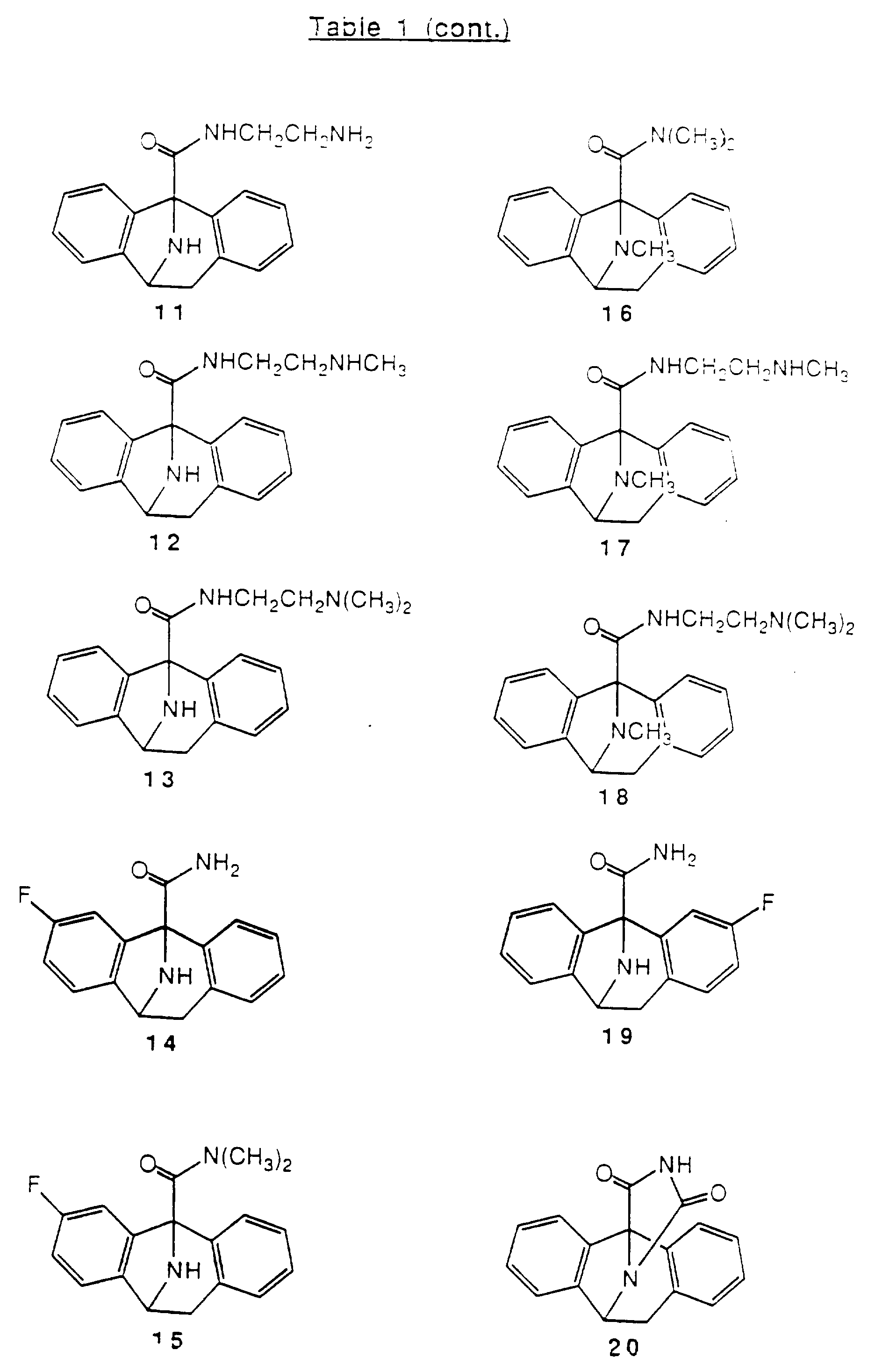

### BIOLOGICAL EVALUATION

Compound 1 was administered intraperotoneally to male CF-1 type mice (20-25 g) witn a saline solution carrier. The dose effect behavior was determined by the administration of six different doses of each compound treating eight mice at each dose. After a period of fifteen minutes, the mice were subjected to corneal application of electroshocks (30 mA at 50 Hz for 0.1s). The ED₅₀ and the 95% confidence intervals of drug wnich eliminated the tonic-extensor component of the convulsion in 50% of the animals was calculatec by the method of Litchfield and Wilcoxon (J. Pharmacol. Exp. Ther, 1949, 96. 99). Drug induced motor toxicity was examined using the inverted screen test which measures the ability of mice to hold onto a screen which has beem turned vertical. Control animals will be able to hold on. Compound 1 showed an ED₅₀ for protection against maximal electroshock induced seizures of 8.9 mg/kg and a TD50 for motor toxicity oi 50-55 mg/kg in mice. Thus compound 1 demonstrates a therapeutic index (TI) of 5.6-6.2.

Administration of compounds within Formula I to humans can be by any technique capable of introducing the compounds into the bloodstream of a human patient, including oral administration, and by intraveneous. intramuscular and subcutaneous injections.

Compounds indicated by prophylactic therapy will preferably be administered in a daily dose generally in the range of 0.1 mg to 100 mg per Kilogram of body weight per day. A more preferred dosage will be in the range of 1.0 to 50 mg per kilogram of body weight. A suitable dose can be administered in suitable subdoses per day.

The active compound is usually administered in a pharmaceutically acceptable formulation, although in some acute-care situations a compound of Formula I may be administered alone. Such formulations may comprise the active compound with one or more pharmaceutically acceptable carriers or diluents. Other therapeutic agents may also be present in the formulation. A pharmaceutically acceptable carrier or diluent provides an appropriate vehicle for delivery of the active compound without undesirable side effects. Delivery of the active compound in such formulations may be by various routes such as oral, nasal, buccal or sublingual, or by parenteral administration such as subcutaneous, intramuscular, intravenous or intradermal routes. Delivery of the active compound may also be through the use of controlled release formulations in subcutaneous implants.

Formulations for oral administration may be in the form of capsules containing the active compound dispersed in a binder such as gelatin or hydroxypropylmethyl cellulose, together with one or more of a lubricant, preservative, surface acting or dispersing agent. Such capsules or tablets may contain controlled release formulation as may be provided in a disposition of active compound in hydroxypropylmethyl cellulose.

Formulations for parental administration may be in the form of aqueous or non-aqueous isotonic sterile injection solutions or suspensions. These solutions or suspensions may be prepared from sterile powders or granules having one or more of the carriers or diluents mentioned for use in the formulations for oral administration.

Although this invention has been described with respect to specific embodiments, the details of these embodiments are not to be construed as limitations. Various equivalents, changes and modifications may be made without departing from the scope of this invention, and it is understood that such equivalent embodiments are part of this invention.

## Claims (Claims for the following Contracting State(s): AT, BE, CH, DE, DK, FR, GB, IT, LI, LU, NL, SE)

1. A compound of formula (I): 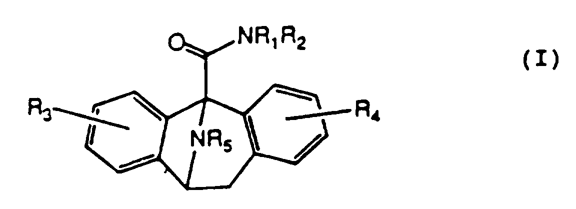 or pharmaceutically acceptable salts of said compound, wherein:
each of R₃ and R₄ is independently selected from hydrogen, halo, linear or branched alkyl groups of from one to ten carbon atoms, alkenyl groups from two to ten carbon atoms, alkynyl groups from two to ten carbon atoms, hydroxy, amino, alkylamino, alkoxy, cyano, nitro, thio, haloalkyl and mercapto; and
R₁, R₂ and R₅ are selected such that either:
(i) each of R₁ and R₂ is independently selected from hydrogen, unsubstituted or substituted linear or branched alkyl groups that contain from one to twenty carbon atoms, unsubstituted or substituted alkenyl groups that contain from two to twenty carbon atoms, unsubstituted or substituted alkynyl groups that contain from two to twenty carbon atoms, unsubstituted or substituted cycloalkyl groups that contain from three to eight carbon atoms, and substituted or unsubstituted cycloalkenyl groups that contain from three to eight carbon atoms, and the substituents of R₁ and R₂ are selected from alkyl, haloalkyl, hydroxyalkyl, alkenyl, oxo, hydroxy, alkoxy, thio, alkoxyalkyl, amino, halo, cyano and mercapto; and
R₅ is selected from, hydrogen, unsubstituted or substituted linear or branched alkyl groups of from one to ten carbon atoms, unsubstituted or substituted alkenyl groups from two to ten carbon atoms, unsubstituted or substituted alkynyl groups of from two to ten carbon atoms, hydroxy, phenyl, haloalkyl, aminoalkyl, 1-phenylmethyl, 2-phenylethyl and alkoxy, and the substituents of R₅ are selected from alkyl, oxo, alkoxy, hydroxy, amino, alkylamino, phenyl, haloalkyl and thio; or
(ii) R₁ and R₂, which are selected as described in (i) above, together with the nitrogen to which R₁ is bound, form a nitrogen-containing heterocyclic group, that has from two to eight carbon atoms; and
R₅ is selected as described in (i) above; or
(iii) R₂ is selected as described in (i) above; R₁, which is selected as described above, and R₅, which is selected as described in (i) above, together with the nitrogens to which each of R₁ and R₅ are bound, form a heterocyclic group that includes the two nitrogen atoms and from two to six carbon atoms
wherein, unless otherwise specified above, the term "alkyl" when used either alone or within other terms such as "haloalkyl" or alkylamino" embraces linear or branched radicals having from one to ten carbon atoms, the term "cycloalkyl" embraces radicals having from three to ten carbon atoms, the term "haloalkyl" embraces radicals wherein one or more of the alkyl carbon atoms is substituted with one or more halogen atoms, the term "alkenyl" embraces linear or branched radicals having from two to ten carbon atoms and containing at least one double bond, the term "alkynyl" embraces linear or branched radicals having from two to ten carbon atoms containing at least one carbon-carbon triple bond, the term "alkoxy" embraces linear or branched oxy-containing radicals having alkyl portions of from one to ten carbon atoms, which may be further substituted with one or more halo atoms, the term "alkylamino" embraces linear or branched nitrogen containing radicals where the nitrogen atom may be substituted with from one to three alkyl radicals of from one to ten carbon atoms.

2. A compound according to claim 1, or a pharmaceutically acceptable salt thereof, wherein:
each of R₁ and R₂ is independently selected from hydrogen, alkyl, alkenyl, alkoxy or phenyl;
each of R₃ and R₄ is independently selected from hydrogen, alkyl, alkenyl, halo, haloalkyl, hydroxy, alkoxy, nitro, cyano, thio, mercapto, amino, alkylamino; and
R₅ is selected from hydrogen, alkyl, alkenyl, haloalkyl, hydroxy, alkoxy, phenyl and aminoalkyl.

3. A compound according to claim 2, or a pharmaceutically acceptable salt thereof, wherein:
each of R₁ and R₂ is independently selected from hydrogen, alkyl, alkenyl, and phenyl; and
each of R₃ and R₄ is independently selected from hydrogen, alkyl, halo, haloalkyl, hydroxy, alkoxy, nitro, amino and alkylamino.

4. A compound according to claim 3, that is 5-aminocarbonyl-5H-dibenzo[a,d]cyclohepten-5,10-imine, or a pharmaceutically acceptable salt thereof.

5. A pharmaceutical composition for treating or preventing epileptic seizures, comprising a pharmaceutically acceptable carrier or diluent and a therapeutically effective concentration of a compound or salt as claimed in any one of the preceding claims, said compound (or salt) and its concentration being effective for the treatment of or prevention of epileptic seizures.

6. A composition for the treatment of drug craving that results from cocaine addiction, comprising an effective concentration of a compound or salt as claimed in any one of claims 1 to 4 and a pharmaceutically effective carrier or diluent, wherein said compound (or salt) and its concentration are effective for reducing said craving for cocaine.

7. For use in the treatment and control of epileptic seizures in mammals, a compound as claimed in any one of claims 1 to 4, or a pharmaceutically acceptable salt thereof.

8. For use in the treatment of drug craving due to cocaine addiction, a compound as claimed in any one of claims 1 to 4, or a pharmaceutically acceptable salt thereof.

9. A process for the preparation of a compound of any one of claims 1 to 4.

10. A process for the preparation of 5-aminocarbonyl-5H-dibenzo[a,d]cyclohepten-5,10-imines as claimed in any one of claims 1 to 4, including the steps of:
(a) converting C5-unsubstituted-10,11-dihydro-5H-dibenzo[a,d]cyclohepten-5,10-imines into their N-tertbutylformamidine derivatives;
(b) treating said N-tertbutylformamidine derivatives with suitable compounds and under suitable conditions to form C5-substituted ethyl ester derivatives;
(c) forming 5-ethoxycarbonyl-10,11-dihydro-5H-dibenzo[a,d]cyclohepten-5,10-imines from said C5-substituted ethyl ester derivatives; and
(d) replacing ester functionality in said 5-ethyoxycarbonyl-10,11-dihydro-5H-dibenzo[a,d]cyclohepten-5,10-imines with an amide group.

## Claims (Claims for the following Contracting State(s): ES)

1. A process for preparing a compound of formula (I): 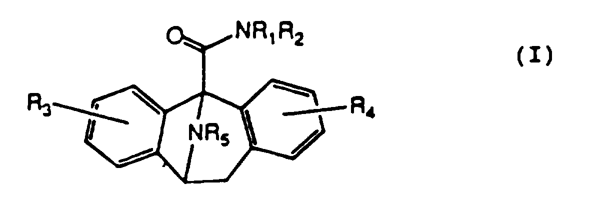 or pharmaceutically acceptable salts of said compound, wherein:
each of R₃ and R₄ is independently selected from hydrogen, halo, linear or branched alkyl groups of from one to ten carbon atoms, alkenyl groups from two to ten carbon atoms, alkynyl groups from two to ten carbon atoms, hydroxy, amino, alkylamino, alkoxy, cyano, nitro, thio, haloalkyl and mercapto; and
R₁, R₂ and R₅ are selected such that either:
(i) each of R₁ and R₂ is independently selected from hydrogen, unsubstituted or substituted linear or branched alkyl groups that contain from one to twenty carbon atoms, unsubstituted or substituted alkenyl groups that contain from two to twenty carbon atoms, unsubstituted or substituted alkynyl groups that contain from two to twenty carbon atoms, unsubstituted or substituted cycloalkyl groups that contain from three to eight carbon atoms, and substituted or unsubstituted cycloalkenyl groups that contain from three to eight carbon atoms, and the substituents of R₁ and R₂ are selected from alkyl, haloalkyl, hydroxyalkyl, alkenyl, oxo, hydroxy, alkoxy, thio, alkoxyalkyl, amino, halo, cyano and mercapto; and
R₅ is selected from hydrogen, unsubstituted or substituted linear or branched alkyl groups of from one to ten carbon atoms, unsubstituted or substituted alkenyl groups from two to ten carbon atoms, unsubstituted or substituted alkynyl groups of from two to ten carbon atoms, hydroxy, phenyl, haloalkyl, aminoalkyl, 1-phenylmethyl, 2-phenylethyl and alkoxy, and the substituents of R₅ are selected from alkyl, oxo, alkoxy, hydroxy, amino, alkylamino, phenyl, haloalkyl and thio; or
(ii) R₁ and R₂, which are selected as described in (i) above, together with the nitrogen to which R₁ is bound, form a nitrogen-containing heterocyclic group, that has from two to eight carbon atoms; and
R₅ is selected as described in (i) above; or
(iii) R₂ is selected as described in (i) above; R₁, which is selected as described above, and R₅, which is selected as described in (i) above, together with the nitrogens to which each of R₁ and R₅ are bound, form a heterocyclic group that includes the two nitrogen atoms and from two to six carbon atoms
wherein, unless otherwise specified above, the term "alkyl" when used either alone or within other terms such as "haloalkyl" or alkylamino" embraces linear or branched radicals having from one to ten carbon atoms, the term "cycloalkyl" embraces radicals having from three to ten carbon atoms, the term "haloalkyl" embraces radicals wherein one or more of the alkyl carbon atoms is substituted with one or more halogen atoms, the term "alkenyl" embraces linear or branched radicals having from two to ten carbon atoms and containing at least one double bond, the term "alkynyl" embraces linear or branched radicals having from two to ten carbon atoms containing at least one carbon-carbon triple bond, the term "alkoxy" embraces linear or branched oxy-containing radicals having alkyl portions of from one to ten carbon atoms, which may be further substituted with one or more halo atoms, the term "alkylamino" embraces linear or branched nitrogen containing radicals where the nitrogen atom may be substituted with from one to three alkyl radicals of from one to ten carbon atoms.

2. A process according to claim 1, wherein:
each of R₁ and R₂ is independently selected from hydrogen, alkyl, alkenyl, alkoxy or phenyl;
each of R₃ and R₄ is independently selected from hydrogen, alkyl, alkenyl, halo, haloalkyl, hydroxy, alkoxy, nitro, cyano, thio, mercapto, amino, alkylamino; and
R₅ is selected from hydrogen, alkyl, alkenyl, haloalkyl, hydroxy, alkoxy, phenyl and aminoalkyl.

3. A process according to claim 2, wherein:
each of R₁ and R₂ is independently selected from hydrogen, alkyl, alkenyl, and phenyl; and
each of R₃ and R₄ is independently selected from hydrogen, alkyl, halo, haloalkyl, hydroxy, alkoxy, nitro, amino and alkylamino.

4. A process according to claim 3, for preparing the following compound: 5-aminocarbonyl-5H-dibenzo[a,d]cyclohepten-5,10-imine, or a pharmaceutically acceptable salt thereof.

5. A process for preparing a pharmaceutical composition for treating or preventing epileptic seizures, comprising combining a pharmaceutically acceptable carrier or diluent with a therapeutically effective concentration of a compound or salt prepared by a process as claimed in any one of the preceding claims, said compound (or salt) and its concentration being effective for the treatment of or prevention of epileptic seizures.

6. A process for preparing a composition for the treatment of drug craving that results from cocaine addiction, comprising combining an effective concentration of a compound or salt prepared by a process as claimed in any one of claims 1 to 4 with a pharmaceutically effective carrier or diluent, wherein said compound (or salt) and its concentration are effective for reducing said craving for cocaine.

7. For use in the treatment and control of epileptic seizures in mammals, a compound prepared by a process as claimed in any one of claims 1 to 4, or a pharmaceutically acceptable salt thereof.

8. For use in the treatment of drug craving due to cocaine addiction, a compound prepared by a process as claimed in any one of claims 1 to 4, or a pharmaceutically acceptable salt thereof.

9. A process for the preparation of 5-aminocarbonyl-5H-dibenzo[a,d]cyclohepten-5,10-imines of formula I as defined in any one of claims 1 to 4, including the steps of:
(a) converting C5-unsubstituted-10,11-dihydro-5H-dibenzo[a,d]cyclohepten-5,10-imines into their N-tertbutylformamidine derivatives;
(b) treating said N-tertbutylformamidine derivatives with suitable compounds and under suitable conditions to form C5-substituted ethyl ester derivatives;
(c) forming 5-ethoxycarbonyl-10,11-dihydro-5H-dibenzo[a,d]cyclohepten-5,10-imines from said C5-substituted ethyl ester derivatives; and
(d) replacing ester functionality in said 5-ethyoxycarbonyl-10,11-dihydro-5H-dibenzo[a,d]cyclohepten-5,10-imines with an amide group.

## Patentansprüche (Patentansprüche für folgende(n) Vertragsstaat(en): AT, BE, CH, DE, DK, FR, GB, IT, LI, LU, NL, SE)

1. Eine Verbindung der Formel (I): 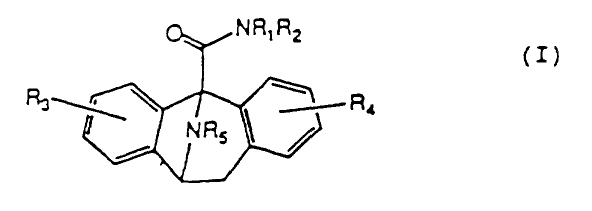 oder pharmazeutisch annehmbare Salze der Verbindung, worin:
R₃ und R₄ jeweils unabhängig aus Wasserstoff, Halogen, linearen oder verzweigten Alkylgruppen mit 1 bis 10 Kohlenstoffatomen, Alkenylgruppen mit 2 bis 10 Kohlenstoffatomen, Alkinylgruppen mit 2 bis 10 Kohlenstoffatomen, Hydroxy, Amino, Alkylamino, Alkoxy, Cyano, Nitro, Thio, Halogenalkyl und Mercapto ausgewählt werden; und
R₁, R₂ und R₅ ausgewählt werden, so daß entweder:
(i) R₁ und R₂ jeweils unabhängig aus Wasserstoff, unsubstituierten oder substituierten linearen oder verzweigten Alkylgruppen, die 1 bis 20 Kohlenstoffatome enthalten, unsubstituierten oder substituierten Alkenylgruppen, die 2 bis 20 Kohlenstoffatome enthalten, unsubstituierten oder substituierten Alkinylgruppen, die 2 bis 20 Kohlenstoffatome enthalten, unsubstituierten oder substituierten Cycloalkylgruppen, die 3 bis 8 Kohlenstoffatome enthalten, und substituierten oder unsubstituierten Cycloalkenylgruppen, die 3 bis 8 Kohlenstoffatome enthalten, ausgewählt werden und die Substituenten R₁ und R₂ aus Alkyl, Halogenalkyl, Hydroxyalkyl, Alkenyl, Oxo, Hydroxy, Alkoxy, Thio, Alkoxyalkyl, Amino, Halogen, Cyano und Mercapto ausgewählt werden; und
R₅ aus Wasserstoff, unsubstituierten oder substituierten linearen oder verzweigten Alkylgruppen mit 1 bis 10 Kohlenstoffatomen, unsubstituierten oder substituierten Alkenylgruppen mit 2 bis 10 Kohlenstoffatomen, unsubstituierten oder substituierten Alkinylgruppen mit 2 bis 10 Kohlenstoffatomen, Hydroxy, Phenyl, Halogenalkyl, Aminoalkyl, 1-Phenylmethyl, 2-Phenylethyl und Alkoxy ausgewählt wird und die Substituenten von R₅ aus Alkyl, Oxo, Alkoxy, Hydroxy, Amino, Alkylamino, Phenyl, Halogenalkyl und Thio ausgewählt werden; oder
(ii) R₁ und R₂, die wie in (i) oben beschrieben ausgewählt werden, zusammen mit dem Stickstoff, an den R₁ gebunden ist, eine Stickstoff-enthaltende heterozyklische Gruppe bilden, die 2 bis 8 Kohlenstoffatome hat; und
R₅ ausgewählt wird wie in (i) oben beschrieben; oder
(iii) R₂ wie oben in (i) beschrieben ausgewählt wird; R₁, das wie oben beschrieben ausgewählt wird, und R₅, das wie oben in (i) beschrieben ausgewählt wird, zusammen mit den Stickstoffen, an die R₁ und R₅ jeweils gebunden werden, eine heterozyklische Gruppe bilden, die die 2 Stickstoffatome und 2 bis 6 Kohlenstoffatome einschließen, worin, wenn oben nicht anders spezifiziert, die Bezeichnung "Alkyl", wenn entweder allein oder in anderen Bezeichnungen wie "Halogenalkyl" oder "Alkylamino" verwendet, lineare oder verzweigte Gruppen mit 1 bis 10 Kohlenstoffatomen umfaßt, die Bezeichnung "Cycloalkyl" Gruppen mit 3 bis 10 Kohlenstoffatomen umfaßt, die Bezeichnung "Halogenalkyl" Gruppen umfaßt, worin ein oder mehrere der Alkyl-Kohlenstoffatome mit einem oder mehreren Halogenatomen substituiert werden, die Bezeichnung "Alkenyl" lineare oder verzweigte Gruppen mit 2 bis 10 Kohlenstoffatomen umfaßt und die wenigstens eine Doppelbindung enthalten, die Bezeichnung "Alkinyl" lineare oder verzweigte Gruppen mit 2 bis 10 Kohlenstoffatomen, die wenigstens eine Kohlenstoff-Kohlenstoff-Dreifachbindung enthalten, umfaßt, die Bezeichnung "Alkoxy" lineare oder verzweigte Oxy-enthaltende Gruppen mit Alkylteilen mit 1 bis 10 Kohlenstoffatomen umfaßt, die mit einem oder mehreren Halogenatomen weiter substituiert sein können, die Bezeichnung "Alkylamino" lineare oder verzweigte Stickstoff-enthaltende Gruppen umfaßt, wo das Stickstoffatom mit 1 bis 3 Alkylgruppen mit 1 bis 10 Kohlenstoffatomen substituiert sein kann.

2. Eine Verbindung gemäß Anspruch 1 oder ein pharmazeutisch annehmbares Salz davon, worin:
R₁ und R₂ jeweils unabhängig aus Wasserstoff, Alkyl, Alkenyl, Alkoxy oder Phenyl ausgewählt werden;
R₃ und R₄ jeweils unabhängig aus Wasserstoff, Alkyl, Alkenyl, Halogen, Halogenalkyl, Hydroxy, Alkoxy, Nitro, Cyano, Thio, Mercapto, Amino, Alkylamino ausgewählt werden; und
R₅ aus Wasserstoff, Alkyl, Alkenyl, Halogenalkyl, Hydroxy, Alkoxy, Phenyl und Aminoalkyl ausgewählt wird.

3. Eine Verbindung gemäß Anspruch 2 oder ein pharmazeutisch annehmbares Salz davon, worin:
R₁ und R₂ unabhängig aus Wasserstoff, Alkyl, Alkenyl und Phenyl ausgewählt werden; und
R₃ und R₄, jeweils unabhängig aus Wasserstoff, Alkyl, Halogen, Halogenalkyl, Hydroxy, Alkoxy, Nitro, Amino und Alkylamino ausgewählt werden.

4. Eine Verbindung gemäß Anspruch 3, die 5-Aminocarbonyl5H-dibenzo[a,d]cyclohepten-5,10-imin oder ein pharmazeutisch annehmbares Salz davon ist.

5. Eine pharmazeutische Zusammensetzung zum Behandeln oder Vorbeugen epileptischer Anfälle, umfassend einen pharmazeutisch annehmbaren Träger oder Verdünnungsmittel und eine therapeutisch wirksame Konzentration einer Verbindung oder eines Salzes wie in einem der vorhergehenden Ansprüche beansprucht, wobei die Verbindung (oder das Salz) und deren Konzentration bei der Behandlung oder Vorbeugung von epileptischen Anfällen wirksam ist.

6. Eine Zusammensetzung zur Behandlung von Drogensucht, die aus Kokainsucht hervorgeht, umfassend eine wirksame Konzentration einer Verbindung oder eines Salzes wie in einem der Ansprüche 1 bis 4 beansprucht und einen pharmazeutisch wirksamen Träger oder Verdünnungsmittel, worin die Verbindung (oder das Salz) und deren Konzentration wirksam beim Verringern der Kokainsucht ist.

7. Für die Verwendung zur Behandlung und Kontrolle von epileptischen Anfallen bei Säugetieren, eine Verbindung wie in einem der Ansprüche 1 bis 4 beansprucht oder ein pharmazeutisch annehmbares Salz davon.

8. Für die Verwendung zur Behandlung von Drogensucht, die auf Kokainsucht zurückzuführen ist, eine Verbindung wie in einem der Ansprüche 1 bis 4 beansprucht oder ein pharmazeutisch annehmbares Salz davon.

9. Ein Verfahren zur Herstellung einer Verbindung nach einem der Ansprüche 1 bis 4.

10. Ein Verfahren zur Herstellung von 5-Aminocarbonyl-5H-dibenzo[a,d]cyclohepten-5,10-iminen wie in einem der Ansprüche 1 bis 4 beansprucht, das die Stufen einschließt:
(a) Umwandeln von an C5-unsubstituierten 10,11-Dihydro-5H-dibenzo[a,d]cyclohepten-5,10-iminen in ihre N-tert-Butylformamidin-Derivate;
(b) Behandeln der N-tert-Butylformamidin-Derivate mit geeigneten Verbindungen und unter geeigneten Bedingungen, um C5-substituierte Ethylester-Derivate zu bilden;
(c) Bilden von 5-Ethoxycarbonyl-10,11-dihydro-5H-dibenzo[a,d]cyclohepten-5,10-iminen aus den C5-substituierten Ethylester-Derivaten; und
(d) Ersetzen der Esterfunktion in den 5-Ethyoxycarbonyl-10,11-dihydro-5H-dibenzo[a,d]cyclohepten-5,10-iminen durch eine Amidgruppe.

## Patentansprüche (Patentansprüche für folgende(n) Vertragsstaat(en): ES)

1. Ein Verfahren zum Herstellen einer Verbindung der Formel (I) : 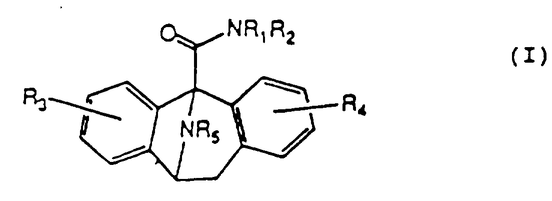 oder pharmazeutisch annehmbarer Salze der Verbindung, worin:
R₃ und R₄ jeweils unabhängig aus Wasserstoff, Halogen, linearen oder verzweigten Alkylgruppen mit 1 bis 10 Kohlenstoffatomen, Alkenylgruppen mit 2 bis 10 Kohlenstoffatomen, Alkinylgruppen mit 2 bis 10 Kohlenstoffatomen, Hydroxy, Amino, Alkylamino, Alkoxy, Cyano, Nitro, Thio, Halogenalkyl und Mercapto ausgewählt werden; und
R₁, R₂ und R₅ ausgewählt werden, so daß entweder:
(i) R₁ und R₂ jeweils unabhängig aus Wasserstoff, unsubstituierten oder substituierten linearen oder verzweigten Alkylgruppen, die 1 bis 20 Kohlenstoffatome enthalten, unsubstituierten oder substituierten Alkenylgruppen, die 2 bis 20 Kohlenstoffatome enthalten, unsubstituierten oder substituierten Alkinylgruppen, die 2 bis 20 Kohlenstoffatome enthalten, unsubstituierten oder substituierten Cycloalkylgruppen, die 3 bis 8 Kohlenstoffatome enthalten, und substituierten oder unsubstituierten Cycloalkenylgruppen, die 3 bis 8 Kohlenstoffatome enthalten, ausgewählt werden und die Substituenten R₁ und R₂ aus Alkyl, Halogenalkyl, Hydroxyalkyl, Alkenyl, Oxo, Hydroxy, Alkoxy, Thio, Alkoxyalkyl, Amino, Halogen, Cyano und Mercapto ausgewählt werden; und
R₅ aus Wasserstoff, unsubstituierten oder substituierten linearen oder verzweigten Alkylgruppen mit 1 bis 10 Kohlenstoffatomen, unsubstituierten oder substituierten Alkenylgruppen mit 2 bis 10 Kohlenstoffatomen, unsubstituierten oder substituierten Alkinylgruppen mit 2 bis 10 Kohlenstoffatomen, Hydroxy, Phenyl, Halogenalkyl, Aminoalkyl, 1-Phenylmethyl, 2-Phenylethyl und Alkoxy ausgewählt wird und die Substituenten von R₅ aus Alkyl, Oxo, Alkoxy, Hydroxy, Amino, Alkylamino, Phenyl, Halogenalkyl und Thio ausgewählt werden; oder
(ii) R₁ und R₂, die wie in (i) oben beschrieben ausgewählt werden, zusammen mit dem Stickstoff, an den R₁ gebunden ist, eine Stickstoff-enthaltende heterozyklische Gruppe bilden, die 2 bis 8 Kohlenstoffatome hat; und
R₅ ausgewählt wird wie in (i) oben beschrieben; oder
(iii) R₂ wie oben in (i) beschrieben ausgewählt wird; R₁, das wie oben beschrieben ausgewählt wird, und R₅, das wie oben in (i) beschrieben ausgewählt wird, zusammen mit den Stickstoffen, an die R₁ und R₅ jeweils gebunden werden, eine heterozyklische Gruppe bilden, die die 2 Stickstoffatome und 2 bis 6 Kohlenstoffatome einschließen, worin, wenn oben nicht anders spezifiziert, die Bezeichnung "Alkyl", wenn entweder allein oder in anderen Bezeichnungen wie "Halogenalkyl" oder "Alkylamino" verwendet, lineare oder verzweigte Gruppen mit 1 bis 10 Kohlenstoffatomen umfaßt, die Bezeichnung "Cycloalkyl" Gruppen mit 3 bis 10 Kohlenstoffatomen umfaßt, die Bezeichnung "Halogenalkyl" Gruppen umfaßt, worin ein oder mehrere der Alkyl-Kohlenstoffatome mit einem oder mehreren Halogenatomen substituiert werden, die Bezeichnung "Alkenyl" lineare oder verzweigte Gruppen mit 2 bis 10 Kohlenstoffatomen umfaßt und die wenigstens eine Doppelbindung enthalten, die Bezeichnung "Alkinyl" lineare oder verzweigte Gruppen mit 2 bis 10 Kohlenstoffatomen, die wenigstens eine Kohlenstoff-Kohlenstoff-Dreifachbindung enthalten, umfaßt, die Bezeichnung "Alkoxy" lineare oder verzweigte Oxy-enthaltende Gruppen mit Alkylteilen mit 1 bis 10 Kohlenstoffatomen umfaßt, die mit einem oder mehreren Halogenatomen weiter substituiert sein können, die Bezeichnung "Alkylamino" lineare oder verzweigte Stickstoff-enthaltende Gruppen umfaßt, wo das Stickstoffatom mit 1 bis 3 Alkylgruppen mit 1 bis 10 Kohlenstoffatomen substituiert sein kann.

2. Ein Verfahren gemäß Anspruch 1, worin:
R₁ und R₂ jeweils unabhängig aus Wasserstoff, Alkyl, Alkenyl, Alkoxy oder Phenyl ausgewählt werden;
R₃ und R₄ jeweils unabhängig aus Wasserstoff, Alkyl, Alkenyl, Halogen, Halogenalkyl, Hydroxy, Alkoxy, Nitro, Cyano, Thio, Mercapto, Amino, Alkylamino ausgewählt werden; und
R₅ aus Wasserstoff, Alkyl, Alkenyl, Halogenalkyl, Hydroxy, Alkoxy, Phenyl und Aminoalkyl ausgewählt wird.

3. Ein Verfahren gemäß Anspruch 2, worin:
R₁ und R₂ jeweils unabhängig aus Wasserstoff, Alkyl, Alkenyl und Phenyl ausgewählt werden; und
R₃ und R₄ jeweils unabhängig aus Wasserstoff, Alkyl, Halogen, Halogenalkyl, Hydroxy, Alkoxy, Nitro, Amino und Alkylamino ausgewählt werden.

4. Ein Verfahren gemäß Anspruch 3 zum Herstellen der folgenden Verbindung: 5-Aminocarbonyl-5H-dibenzo[a,d]cyclohepten-5,10-imin oder ein pharmazeutisch annehmbares Salz davon.

5. Ein Verfahren zum Herstellen einer pharmazeutischen Zusammensetzung zum Behandeln oder Vorbeugen epileptischer Anfälle, umfassend ein Verbinden eines pharmazeutisch annehmbaren Trägers oder Verdünnungsmittels mit einer therapeutisch wirksamen Konzentration einer Verbindung oder eines Salzes, hergestellt durch ein Verfahren wie in einem der vorhergehenden Ansprüche beansprucht, wobei die Verbindung (oder das Salz) und deren Konzentration bei der Behandlung oder Vorbeugung von epileptischen Anfällen wirksam ist.

6. Ein Verfahren zum Herstellen einer Zusammensetzung zur Behandlung von Drogensucht, die aus Kokainsucht hervorgeht, umfassend ein Verbinden einer wirksamen Konzentration einer Verbindung oder eines Salzes, hergestellt durch ein Verfahren wie in einem der Ansprüche 1 bis 4 beansprucht mit einem pharmazeutisch wirksamen Träger oder Verdünnungsmittel, worin die Verbindung (oder das Salz) und deren Konzentration wirksam beim Verringern der Kokainsucht ist.

7. Für die Verwendung zur Behandlung und Kontrolle von epileptischen Anfällen bei Säugetieren, eine Verbindung, hergestellt durch ein Verfahren wie in einem der Ansprüche 1 bis 4 beansprucht oder ein pharmazeutisch annehmbares Salz davon.

8. Für die Verwendung zur Behandlung von Drogensucht, die auf Kokainsucht zurückzuführen ist, eine Verbindung, hergestellt durch ein Verfahren wie in einem der Ansprüche 1 bis 4 beansprucht oder ein pharmazeutisch annehmbares Salz davon.

9. Ein Verfahren zur Herstellung von 5-Aminocarbonyl-5H-dibenzo[a,d]cyclohepten-5,10-iminen der Formel I wie in einem der Ansprüche 1 bis 4 definiert, das die Stufen einschließt:
(a) Umwandeln von C5-unsubstituierten 10,11-Dihydro-5H-dibenzo[a,d]cyclohepten-5,10-iminen in ihre N-tert-Butylformamidin-Derivate;
(b) Behandeln der N-tert-Butylformamidin-Derivate mit geeigneten Verbindungen und unter geeigneten Bedingungen, um C5-substituierte Ethylester-Derivate zu bilden;
(c) Bilden von 5-Ethoxycarbonyl-10,11-dihydro-5H-dibenzo[a,d]cyclohepten-5,10-iminen aus den C5-substituierten Ethylester-Derivaten; und
(d) Ersetzen der Esterfunktion in den 5-Ethyoxycarbonyl-10,11-dihydro-5H-dibenzo[a,d]cyclohepten-5,10-iminen durch eine Amidgruppe.

## Revendications (Revendications pour l'(les) Etat(s) contractant(s) suivant(s): AT, BE, CH, DE, DK, FR, GB, IT, LI, LU, NL, SE.)

1. Composé de formule (I) : 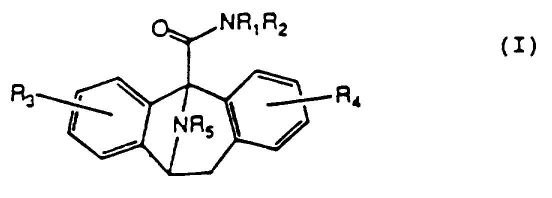 où les sels phannaceutiquement acceptables dudit composé, où :
chaque groupe R₃ et R₄ est choisi indépendamment parmi un hydrogène, halogène, les groupes alkyles linéaires ou ramifiés de 1 à 10 atomes de carbone, les groupes alcényles de 2 à 10 atomes de carbone, les groupes alcynyles de 2 à 10 atomes de carbone, les groupes hydroxy, amino, alkylamino, alcoxy, cyano, nitro, thio, haloalkyle et mercapto ; et
R₁, R₂ et R₅ sont choisis de telle manière que soit :
(i) on choisit indépendamment chacun des groupes R₁ et R₂ parmi un hydrogène, des groupes alkyles linéaires ou ramifiés, substitués ou non substitués qui contiennent de 1 à 20 atomes de carbone, les groupes alcényles non substitués ou substitués qui contiennent de 2 à 20 atomes de carbone, les groupes alcynyles substitués ou non substitués qui contiennent de 2 à 20 atomes de carbone, les groupes cycloalkyles substitués ou non substitués qui contiennent de 3 à 8 atomes de carbone, les groupes cycloalcényles substitués ou non substitués qui contiennent de 3 à 8 atomes de carbone, et les substituants de R₁ et R₂ sont choisis parmi les alkyle, haloalkyle, hydroxyalkyle, alcényle, oxo, hydroxy, alcoxy, thio, alcoxyalkyle, amino, halo, cyano et mercapto ; et
on choisit R₅ parmi un hydrogène, les groupes alkyles linéaires ou ramifiés non substitués ou substitués de 1 à 10 atomes de carbone, les groupes alcényles substitués ou non substitués de 2 à 10 atomes de carbone, les groupes alcynyles substitués ou non substitués de 2 à 10 atomes de carbone, les groupes hydroxy, phényle, haloalkyle, aminoalkyle, 1-phénylméthyle, 2-phényléthyle et alcoxy, et on choisit les substituants de R₅ parmi les alkyle, oxo, alcoxy, hydroxy, amino, alkylamino, phényle, haloalkyle et thio ; soit
(ii) R₁ et R₂, qu'on a sélectionné comme c'est décrit au paragraphe (i) ci-dessus, avec un atome d'azote auquel R₁ est fixé, forment un groupe hétérocyclique contenant de l'azote qui a de 2 à 8 atomes de carbone ; et
R₅ est choisi comme c'est décrit en (i) ci-dessus ; soit
(iii) R₂ est choisi comme c'est décrit en (i) ci-dessus ; R₁ qui est choisi comme c'est décrit ci-dessus, et R₅ qui est choisi comme c'est décrit en (i) ci-dessus, avec les atomes d'azote auxquels chaque R₁ et R₅ est fixé, forment un groupe hétérocyclique qui comprend les deux atomes d'azote et de 2 à environ 6 atomes de carbone, où sauf indication contraire, le terme "alkyle" quand on l'utilise soit seul, soit en présence d'autres termes tels que "haloalkyle" ou "alkylamino" comprend des radicaux linéaires ou ramifiés ayant de 1 à 10 atomes de carbone, le terme "cycloalkyle" comprend des radicaux ayant de 3 à 10 atomes de carbone, le terme "haloalkyle" comprend des radicaux où un ou plusieurs des atomes de carbone d'alkyle sont substitués par un ou plusieurs atomes d'halogène, le terme "alcényle" comprend des radicaux linéaires ou ramifiés ayant de 2 à 10 atomes de carbone et contenant au moins une double liaison, le terme "alcynyle" comprend des radicaux linéaires ou ramifiés ayant de 2 à 10 atomes de carbone contenant au moins une triple liaison carbone-carbone, le terme "alcoxy" comprend des radicaux linéaires ou ramifiés contenant de l'oxygène ayant des parties alkyles de 1 à 10 atomes de carbone, qu'on peut en outre substituer par un ou plusieurs atomes d'halogènes, le terme "alkylamino" comprend des radicaux linéaires ou ramifiés contenant de l'azote où l'atome d'azote peut être substitué avec de 1 à 3 radicaux alkyles, de 1 à 10 atomes de carbone.

2. Composé selon la revendication 1, ou un de ses sels pharmaceutiquement acceptable, où :
chaque R₁ et R₂ est choisi indépendamment parmi les hydrogène, alkyle, alcényle, alcoxy ou phényle :
chaque groupe R₃ et R₄ est choisi indépendamment parmi les hydrogène, alkyle, alcényle, halo, haloalkyle, hydroxy, alcoxy, nitro, cyano, thio, mercapto, amino, alkylamino ; et
R₅ est choisi parmi les hydrogène, alkyle, alcényle, haloalkyle, hydroxy, alcoxy, phényle et aminoalkyle.

3. Composé selon la revendication 2, ou un de ses sels pharmaceutiquement acceptable, où :
chaque groupe R₁ et R₂ est choisi indépendamment parmi les hydrogène, alkyle, alcényle et phényle ; et
chaque groupe R₃ et R₄ est choisi indépendamment parmi les hydrogène, alkyle, halo, haloalkyle, hydroxy, alcoxy, nitro, amino et alkylamino.

4. Composé selon la revendication 3, qui est la 5-aminocarbonyl-5H-dibenzo[a,d]cyclohepten-5,10-imine, ou un de ses sels pharmaceutiquement acceptable.

5. Composition pharmaceutique pour traiter ou prévenir les crises épileptiques, comprenant un véhicule ou diluant pharmaceutiquement acceptable et une concentration thérapeutiquement efficace d'un composé ou d'un sel selon l'une quelconque des revendications précédentes, ledit composé (ou sel) et sa concentration étant efficaces au traitement ou à la prévention des crises épileptiques.

6. Composition pour traiter l'appétance toxicomaniaque qui résulte de la dépendance à la cocaïne, comprenant une concentration efficace d'un composé ou d'un sel selon une quelconque des revendications 1 à 4 et un véhicule ou diluant pharmaceutiquement efficace, où ledit composé (ou sel) et sa concentration sont efficaces pour réduire ladite appétance pour la cocaïne.

7. Composé selon l'une quelconque des revendications 1 à 4, ou un de ses sels pharmaceutiquement acceptable pour l'utilisation dans le traitement et la maîtrise des crises épileptiques chez les mammifères.

8. Composé selon l'une quelconque des revendications 1 à 4 ou un de ses sels pharmaceutiquement acceptable pour l'utilisation dans le traitement de l'appétance toxicomaniaque due à la dépendance à la cocaïne.

9. Procédé de préparation d'un composé selon l'une quelconque des revendications 1 à 4.

10. Procédé de préparation des 5-aminocarbonyl-5H-dibenzo[a,d]cyclohepten-5,10-imines selon l'une quelconque des revendications 1 à 4, comprenant les étapes de :
(a) convertir les C5-non substituées-10,11-dihydro-5H-dibenzo[a,d]cyclohepten-5,10-imines en leurs dérivés de N-tertbutylformamidine ;
(b) traiter lesdits dérivés de N-tertbutylformamidine avec des composés appropriés et dans des conditions convenables pour former les dérivés esters éthyliques C5-substitués ;
(c)former les 5-éthoxycarbonyl-10,11-dihydro-5H-dibenzo[a,d]cyclohepten-5,10-imines à partir desdits dérivés esters éthyliques C5-substitués ; et
(d) remplacer ladite fonctionnalité ester dans lesdites 5-éthoxy-carbonyl-10,11-dihydro-5H-dibenzo[a,d]cyclohepten-5,10-imines par un groupe amide.

## Revendications (Revendications pour l'(les) Etat(s) contractant(s) suivant(s): ES)

1. Procédé de préparation d'un composé de formule (I) : 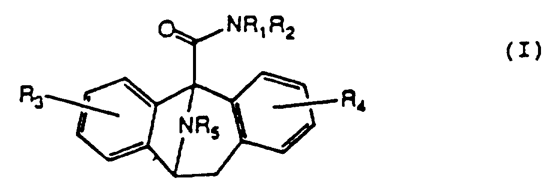 ou les sels pharmaceutiquement acceptables dudit composé, où :
chaque groupe R₃ et R₄ est choisi indépendamment parmi un hydrogène, halogène, les groupes alkyles linéaires ou ramifiés de 1 à 10 atomes de carbone, les groupes alcényles de 2 à 10 atomes de carbone, les groupes alcynyles de 2 à 10 atomes de carbone, les groupes hydroxy, amino, alkylamino, alcoxy, cyano, nitro, thio, haloalkyle et mercapto ; et
R₁, R₂ et R₅ sont choisis de telle manière que soit :
(i) on choisit indépendamment chacun des groupes R₁ et R₂ parmi un hydrogène, des groupes alkyles linéaires ou ramifiés, substitués ou non substitués qui contiennent de 1 à 20 atomes de carbone, les groupes alcényles non substitués ou substitués qui contiennent de 2 à 20 atomes de carbone, les groupes alcynyles substitués ou non substitués qui contiennent de 2 à 20 atomes de carbone, les groupes cycloalkyles substitués ou non substitués qui contiennent de 3 à 8 atomes de carbone, les groupes cycloalcényles substitués ou non substitués qui contiennent de 3 à 8 atomes de carbone, et les substituants de R₁ et R₂ sont choisis parmi les alkyle, haloalkyle, hydroxyalkyle, alcényle, oxo, hydroxy, alcoxy, thio, alcoxyalkyle, amino, halo, cyano et mercapto ; et
on choisit R₅ parmi un hydrogène, les groupes alkyles linéaires ou ramifiés non substitués ou substitués de 1 à 10 atomes de carbone, les groupes alcényles substitués ou non substitués de 2 à 10 atomes de carbone, les groupes alcynyles substitués ou non substitués de 2 à 10 atomes de carbone, les groupes hydroxy, phényle, haloalkyle, aminoalkyle, 1-phénylméthyle, 2-phényléthyle et alcoxy, et on choisit les substituants de R₅ parmi les alkyle, oxo, alcoxy, hydroxy, amino, alkylamino, phényle, haloalkyle et thio ; soit
(ii) R₁ et R₂, qu'on a sélectionné comme c'est décrit au paragraphe (i) ci-dessus, avec un atome d'azote auquel R₁ est fixé, forment un groupe hétérocyclique contenant de l'azote qui a de 2 à 8 atomes de carbone ; et
R₅ est choisi comme c'est décrit en (i) ci-dessus ; soit
(iii) R₂ est choisi comme c'est décrit en (i) ci-dessus ; R₁ qui est choisi comme c'est décrit ci-dessus, et R₅ qui est choisi comme c'est décrit en (i) ci-dessus, avec les atomes d'azote auxquels chaque R₁ et R₅ est fixé, forment un groupe hétérocyclique qui comprend les deux atomes d'azote et de 2 à environ 6 atomes de carbone, où sauf indication contraire, le terme "alkyle" quand on l'utilise soit seul, soit en présence d'autres termes tels que "haloalkyle" ou "alkylamino" comprend des radicaux linéaires ou ramifiés ayant de 1 à 10 atomes de carbone, le terme "cycloalkyle" comprend des radicaux ayant de 3 à 10 atomes de carbone, le terme "haloalkyle" comprend des radicaux où un ou plusieurs des atomes de carbone d'alkyle sont substitués par un ou plusieurs atomes d'halogène, le terme "alcényle" comprend des radicaux linéaires ou ramifiés ayant de 2 à 10 atomes de carbone et contenant au moins une double liaison, le terme "alcynyle" comprend des radicaux linéaires ou ramifiés ayant de 2 à 10 atomes de carbone contenant au moins une triple liaison carbone-carbone, le terme "alcoxy" comprend des radicaux linéaires ou ramifiés contenant de l'oxygène ayant des parties alkyles de 1 à 10 atomes de carbone, qu'on peut en outre substituer par un ou plusieurs atomes d'halogènes, le terme "alkylamino" comprend des radicaux linéaires ou ramifiés contenant de l'azote où l'atome d'azote peut être substitué avec de 1 à 3 radicaux alkyles, de 1 à 10 atomes de carbone.

2. Procédé selon la revendication 1, où :
chaque R₁ et R₂ est choisi indépendamment parmi les hydrogène, alkyle, alcényle, alcoxy ou phényle :
chaque groupe R₃ et R₄ est choisi indépendamment parmi les hydrogène, alkyle, alcényle, halo, haloalkyle, hydroxy, alcoxy, nitro, cyano, thio, mercapto, amino, alkylamino ; et
R₅ est choisi parmi les hydrogène, alkyle, alcényle, haloalkyle, hydroxy, alcoxy, phényle et aminoalkyle.

3. Procédé selon la revendication 2, où :
chaque groupe R₁ et R₂ est choisi indépendamment parmi les hydrogène, alkyle, alcényle et phényle ; et
chaque groupe R₃ et R₄ est choisi indépendamment parmi les hydrogène, alkyle, halo, haloalkyle, hydroxy, alcoxy, nitro, amino et alkylamino.

4. Procédé selon la revendication 3 de préparation du composé suivant : 5-aminocarbonyl-5H-dibenzo[a,d]cyclohepten-5,10-imine, ou un de ses sels pharmaceutiquement acceptable.

5. Procédé de préparation d'une composition pharmaceutique pour traiter ou prévenir les crises épileptiques, comprenant de combiner un véhicule ou diluant pharmaceutiquement acceptable et une concentration thérapeutiquement efficace d'un composé ou d'un sel préparé par un procédé selon l'une quelconque des revendications précédentes, ledit composé (ou sel) et sa concentration étant efficaces au traitement ou à la prévention des crises épileptiques.

6. Procédé de préparation d'une composition pour traiter l'appétance toxicomaniaque qui résulte de la dépendance à la cocaïne, comprenant de combiner une concentration efficace d'un composé ou d'un sel préparé selon une quelconque des revendications 1 à 4 et un véhicule ou diluant pharmaceutiquement efficace, où ledit composé (ou sel) et sa concentration sont efficaces pour réduire ladite appétance pour la cocaïne.

7. Composé préparé par un procédé selon l'une quelconque des revendications 1 à 4, ou un de ses sels pharmaceutiquement acceptable pour l'utilisation dans le traitement et la maîtrise des crises épileptiques chez les mammifères.

8. Composé préparé par un procédé selon l'une quelconque des revendications 1 à 4 d'un de ses sels pharmaceutiquement acceptable pour l'utilisation dans le traitement de l'appétance toxicomaniaque due à la dépendance à la cocaïne.

9. Procédé de préparation des 5-aminocarbonyl-5H-dibenzo[a,d]cyclohepten-5,10-imines selon l'une quelconque des revendications 1 à 4, comprenant les étapes de :
(a) convertir les C5-non substituées-10,11-dihydro-5H-dibenzo[a,d]cyclohepten-5,10-imines en leurs dérivés de N-tertbutylformamidine;
(b) traiter lesdits dérivés de N-tertbutylformamidine avec des composés appropriés et dans des conditions convenables pour former les dérivés esters éthyliques CS-substitués;
(c) former les 5-éthoxycarbonyl-10,11-dihydro-5H-dibenzo[a,d]cyclohepten-5,10-imines à partir desdits dérivés esters éthyliques C5-substitués ; et
(d) remplacer ladite fonctionnalité ester dans lesdites 5-éthoxycarbonyl-10,11-dihydro-5H-dibenzo[a,d]cyclohepten-5,10-imines par un groupe amide.
